# FASCICULE DE BREVET EUROPEEN

(11) **EP 3 975 886 B1**
(45) Date de publication et mention de la délivrance du brevet: **02.07.2025**
(21) Numéro de dépôt: 20737265.7
(22) Date de dépôt: 20.05.2020
(51) Int. Cl.: A61B 17/32, A61B 17/34

(54) **INSTRUMENT DE TRAITEMENT CHIRURGICAL**
CHIRURGISCHES BEHANDLUNGSINSTRUMENT
SURGICAL TREATMENT INSTRUMENT

(30) Priorité: 24.05.2019 FR 1905492
(43) Date de publication de la demande: 06.04.2022
(73) Titulaire: Innoprod Medical, 31830 Plaisance-du-Touch (FR)
(72) Inventeur: LEGAY, Philippe Alain Lucien Fernand, 76530 Yville-sur-Seine (FR); PETROVER, David, 75017 Paris (FR); PEYRE, Frédéric, 31530 Lasserre (FR)
(74) Mandataire: Icosa
(86) Numéro de dépôt international: PCT/FR2020/050850
(87) Numéro de publication internationale: WO 2020/240121

(56) Documents cités:
- US-A- 5 269 796
- US-A- 5 569 283
- US-A1- 2008 009 848
- US-A1- 2016 113 671

## Description

L'invention a trait à un instrument de traitement chirurgical, notamment pour des interventions comprenant la section d'un tissu fibreux telles que des ténolyses, ténotomies ou neurolyses. L'invention a également trait à un ensemble comprenant un instrument de traitement chirurgical associé à une canule d'insertion et/ou une seringue. De plus, la divulgation trait à une canule d'insertion (non comprise telle qu'elle dans la portée des revendications) pour un instrument de traitement chirurgical, et à une méthode de traitement chirurgical (non comprise dans la portée des revendications), comprenant notamment la section d'un tissu fibreux, telle qu'une ténolyse, une ténotomie ou une neurolyse, à l'aide d'un instrument de traitement chirurgical.

De manière connue, une ténolyse est une intervention chirurgicale comprenant la section d'une adhérence constituée au niveau d'un tendon ; une ténotomie est une intervention chirurgicale comprenant la section d'un tendon ; une neurolyse est une intervention chirurgicale comprenant la section d'une adhérence comprimant un nerf. Un exemple de pathologie dont le traitement chirurgical met en jeu une ténolyse est le doigt à ressaut. Plus précisément, le traitement chirurgical du doigt à ressaut comprend la section de la poulie, i.e. la gaine, qui entoure le tendon fléchisseur, de manière à permettre la libération du tendon fléchisseur par rapport à la poulie. Un exemple de pathologie dont le traitement chirurgical met en jeu une neurolyse est le syndrome du canal carpien. Plus précisément, le traitement chirurgical du syndrome du canal carpien comprend la section du ligament annulaire antérieur du carpe, de manière à permettre la libération du nerf médian par rapport aux structures qui le compriment. Des interventions comprenant la section d'un tissu fibreux telles que des ténolyses, ténotomies ou neurolyses, peuvent être mises en œuvre pour le traitement de différentes parties du corps, notamment pour la main, l'épaule, le genou, la cheville, le pied.

Lors d'une ténolyse, ténotomie ou neurolyse, il est classique d'effectuer la section du tissu fibreux à l'aide d'un instrument de type rétro-couteau, ayant un crochet tranchant. Le crochet de l'instrument est d'abord introduit à l'avant du tissu fibreux, puis l'instrument est soumis à un mouvement de translation de l'avant vers l'arrière du tissu fibreux en direction d'une incision d'entrée, de manière à sectionner le tissu fibreux. US 2016/113671 A1 divulgue un tel instrument.

Actuellement, les ténolyses, ténotomies et neurolyses sont pratiquées :
- soit à ciel ouvert, en exécutant une large incision impliquant des suites opératoires relativement longues et douloureuses, nécessitant un arrêt d'activité important ;
- soit par endoscopie, en effectuant une incision plus réduite et en utilisant des instruments différents pour l'étape d'introduction, qui se pratique à l'aveugle, puis pour l'étape de section du tissu fibreux, qui se pratique avec une vision limitée à l'optique de l'endoscope, les suites opératoires restant relativement longues et douloureuses, nécessitant encore un arrêt d'activité important.

C'est à ces inconvénients qu'entend plus particulièrement remédier l'invention en proposant un instrument et une méthode de traitement chirurgical (non comprise dans la portée des revendications) permettant d'effectuer une intervention telle qu'une ténolyse, une ténotomie ou une neurolyse avec un instrument unique, en un seul geste et d'une seule main, étant ainsi compatible avec un monitoring à l'aide d'un appareil tenu dans l'autre main, tel qu'une sonde échographique, tout en limitant les douleurs post-opératoires et la durée d'arrêt d'activité.

A cet effet, selon un aspect, l'invention a pour objet un instrument de traitement chirurgical selon la revendication 1, notamment pour ténolyses, ténotomies ou neurolyses, comprenant un corps principal et une partie distale, où la partie distale comporte une portion courbe formant un crochet et est munie d'une lame de coupe du côté intérieur du crochet, le corps principal comportant une lumière longitudinale de passage de fluide depuis une extrémité proximale vers une extrémité distale du corps principal, le corps principal comportant en outre un coude de concavité tournée du même côté que le crochet.

L'instrument de traitement chirurgical revendiqué est tel que le corps principal comporte, au voisinage de son extrémité distale, un canal d'injection qui est en connexion fluidique avec la lumière par l'intermédiaire d'une chambre de surpression. Le canal d'injection est destiné à diriger un jet de fluide au-dessus du crochet et la chambre de surpression est configurée pour fournir du fluide sous pression au niveau du canal d'injection.

La lumière longitudinale du corps principal permet, lors d'une intervention chirurgicale impliquant la section d'un tissu fibreux, d'injecter un fluide en surpression à l'avant du crochet de manière à écarter les parties molles et produire une hydro-dissection lors de la progression de la partie distale de l'instrument sous le tissu fibreux. Par ailleurs, le coude du corps principal fournit un appui permettant d'imprimer une rotation contrôlée à l'instrument, autour du coude formant un axe de rotation, de manière à relever le crochet en direction du tissu fibreux pour l'accrocher, avant de le sectionner par un mouvement de retrait de l'instrument. Ainsi, un instrument de traitement chirurgical selon l'invention, dont le corps principal présente à la fois une lumière longitudinale de passage de fluide et un coude de concavité tournée du même côté que le crochet, permet d'effectuer avec le même instrument, tenu d'une seule main, et en un seul geste, à la fois l'étape d'avancée du crochet sous le tissu fibreux jusqu'à ce qu'il le dépasse, grâce à l'hydro-dissection, et l'étape de crochetage et section du tissu fibreux, grâce au guidage en rotation assuré par le coude du corps principal, depuis la position du crochet dépassant au-delà du tissu fibreux, ce qui garantit une venue en prise contrôlée du crochet vis-à-vis du tissu fibreux avant d'initier le mouvement de retrait générant la section du tissu fibreux.

Le ou chaque canal d'injection est configuré pour diriger un jet de fluide radialement vers l'extérieur par rapport au crochet de la partie distale.

Le corps principal comporte, au voisinage de son extrémité distale, au moins un canal d'injection en connexion fluidique avec la lumière du corps principal, le ou chaque canal d'injection étant configuré pour diriger un jet de fluide radialement vers l'extérieur par rapport au crochet de la partie distale. Cet agencement permet de garantir que les jets de fluide en surpression émis à partir du ou de chaque canal d'injection devancent le crochet, de manière à pratiquer l'hydro-dissection lors de l'étape d'avancée du crochet. De préférence, afin de ne pas gêner l'avancée du crochet sous le tissu fibreux, le ou chaque canal d'injection est configuré pour diriger un jet de fluide radialement vers l'extérieur par rapport au crochet sans faire saillie radialement vers l'extérieur par rapport à la partie distale.

Selon un mode de réalisation préféré, la chambre de surpression est de section transversale supérieure à la section transversale de la lumière du corps principal et à la section transversale de chaque canal d'injection.

Selon une caractéristique, le corps principal comporte, entre la lumière du corps principal et le ou chaque canal d'injection, ladite chambre de surpression de section transversale supérieure à la section transversale de la lumière du corps principal et à la section transversale de chaque canal d'injection. Grâce à la section plus importante de la chambre de surpression par rapport à celles du ou de chaque canal d'injection et de la lumière du corps principal, la chambre de surpression garde toujours un volume de fluide élevé, de sorte qu'elle est efficace instantanément pour fournir du fluide sous pression au niveau de chaque canal d'injection et pratiquer l'hydro-dissection. En particulier, la chambre de surpression permet de projeter du fluide sous pression au niveau du ou de chaque canal d'injection sans temps de latence entre, d'une part, l'action d'un opérateur sur un dispositif de distribution de fluide, tel qu'une seringue, connecté avec la lumière du corps principal au niveau de l'extrémité proximale de l'instrument et, d'autre part, l'éjection de fluide au niveau du ou de chaque canal d'injection. De plus, le volume plus élevé de la chambre de surpression permet de limiter les fuites de fluide au niveau du ou de chaque canal d'injection.

Selon une caractéristique, le ou chaque canal d'injection est défini dans le prolongement d'une partie supérieure de la lumière du corps principal, et d'une partie supérieure de la chambre de surpression lorsqu'elle est présente, de manière à diriger un jet de fluide au-dessus du crochet. On entend ici par « partie supérieure » de la lumière du corps principal ou de la chambre de surpression, une partie qui fait face à l'extrémité libre du crochet, alors qu'une « partie inférieure » de la lumière du corps principal ou de la chambre de surpression est une partie qui fait face à la base du crochet. Selon un agencement avantageux, la lumière du corps principal, et la chambre de surpression lorsqu'elle est présente, sont disposées en face de l'ouverture du crochet en s'étendant radialement depuis la base du crochet jusqu'à l'extrémité libre du crochet, alors que le ou chaque canal d'injection est disposé en face de l'ouverture du crochet en s'étendant radialement depuis une portion médiane du crochet jusqu'à l'extrémité libre du crochet. La redirection des jets de fluide au-dessus du crochet peut ainsi être obtenue sans que le ou chaque canal d'injection fasse saillie radialement vers l'extérieur par rapport à la partie distale, ce qui permet de ne pas perturber la progression du crochet lors de son avancée sous le tissu fibreux.

Selon une caractéristique, pour le ou chaque canal d'injection, le rapport de la section transversale du canal d'injection sur la section transversale de la chambre de surpression est inférieur ou égal à 0,5, de préférence compris entre 0,45 et 0,5. Un tel rapport de sections entre le ou chaque canal d'injection et la chambre de surpression permet de créer une surpression au niveau de chaque canal d'injection, de telle sorte que les jets de fluide propulsés à partir de chaque canal d'injection sont suffisamment puissants pour écarter les parties molles et permettre l'hydro-dissection lors de l'étape d'avancée du crochet.

Selon une caractéristique, le rapport de la section transversale de la lumière du corps principal sur la section transversale de la chambre de surpression est inférieur ou égal à 0,7, de préférence compris entre 0,6 et 0,7. Un tel rapport de sections entre la lumière du corps principal et la chambre de surpression permet de maintenir un volume de fluide élevé dans la chambre de surpression, ce qui a le double avantage de limiter les fuites de fluide au niveau du ou de chaque canal d'injection et de garantir une réactivité optimale de l'instrument pour fournir du fluide sous pression au niveau du ou de chaque canal d'injection, sans temps de latence.

Selon une caractéristique, le coude définit du côté concave un angle compris entre 90° et 160°, de préférence de l'ordre de 120°, entre une portion du corps principal en amont du coude et une portion du corps principal en aval du coude. Un tel angle entre les portions en amont et en aval du coude est optimal pour permettre au praticien de réaliser, de manière aisée et fiable, la rotation de l'instrument autour du coude depuis la position du crochet dépassant au-delà du tissu fibreux, de sorte que le crochet vient crocheter le tissu fibreux directement par le biais de cette rotation.

Selon une caractéristique, la lame de coupe du crochet comporte une partie de lame intérieure, située à l'intérieur du crochet, et une partie de lame amont, située en amont du crochet, la partie de lame amont étant inclinée vers l'intérieur du crochet, notamment selon un angle compris entre 5° et 30°, par rapport à l'axe longitudinal de la partie distale. Cette inclinaison de la partie de lame amont permet, une fois le tissu fibreux crocheté à l'aide du crochet, d'avoir la partie de lame amont orientée transversalement, c'est-à-dire en biais, par rapport au tissu et en prise avec celui-ci. Il est ainsi possible d'opérer une découpe du tissu fibreux non seulement au moyen de la partie de lame intérieure, mais aussi au moyen de la partie de lame amont lors du mouvement de retrait de l'instrument, ce qui n'est pas le cas avec une partie de lame amont parallèle à l'axe longitudinal de la partie distale ou inclinée vers l'extérieur du crochet. Il en résulte une meilleure efficacité de coupe de l'instrument et un effort réduit à fournir par le praticien pour sectionner le tissu fibreux lors du mouvement de retrait de l'instrument.

L'inclinaison de la partie de lame amont est particulièrement efficace en association avec le coude du corps principal. En effet, grâce au coude du corps principal, l'instrument selon l'invention permet d'imposer une inclinaison fixe de la partie distale par rapport au tissu fibreux lors du mouvement de retrait, ce qui permet de garantir une prise optimale du tissu fibreux entre la partie de lame intérieure et la partie de lame amont.

La longueur cumulée de la portion du corps principal en aval du coude et de la partie distale est généralement fixée pour une application donnée. Par exemple, dans le cas d'interventions au niveau du poignet ou du doigt, notamment dans le cadre du traitement du syndrome du canal carpien ou du doigt à ressaut, la longueur cumulée de la portion du corps principal en aval du coude et de la partie distale est typiquement comprise entre 15 mm et 80 mm.

Selon une caractéristique, la longueur de la portion du corps principal en amont du coude peut être modulée de manière à ajuster le bras de levier, et donc la force à appliquer par le praticien à l'aide de l'instrument, pour sectionner le tissu fibreux lors du mouvement de retrait de l'instrument. En particulier, dans le cas d'interventions au niveau du poignet ou du doigt, notamment dans le cadre du traitement du syndrome du canal carpien ou du doigt à ressaut, la longueur de la portion du corps principal en amont du coude est typiquement comprise entre 10 mm et 100 mm.

Selon une caractéristique, l'instrument de traitement chirurgical comprend un embout configuré pour coopérer avec une seringue, de manière à mettre le corps de seringue en connexion fluidique avec la lumière du corps principal au niveau de l'extrémité proximale du corps principal. Il est ainsi possible d'injecter un fluide en surpression depuis le corps de seringue vers la partie distale de l'instrument, en agissant sur le piston de la seringue, pour pratiquer l'hydro-dissection à l'avant du crochet lors de l'étape d'avancée du crochet sous le tissu fibreux. De manière avantageuse, l'embout de l'instrument de traitement chirurgical est un embout mâle ou femelle, notamment de type Luer Lock, configuré pour coopérer avec un embout complémentaire femelle ou mâle, notamment de type Luer Lock, de la seringue.

Selon une caractéristique pas comprise dans la portée des revendications, la rotation de l'instrument de traitement chirurgical autour du coude est actionnée par le praticien en agissant sur une seringue qui est liée à l'extrémité proximale de l'instrument et forme une poignée d'actionnement. De manière avantageuse, le praticien peut alors ne pas changer sa prise sur le corps de seringue entre l'étape d'avancée du crochet sous le tissu fibreux et l'étape de crochetage et section du tissu fibreux : en particulier, dans l'étape d'avancée du crochet sous le tissu fibreux, le praticien peut tenir le corps de seringue d'une main et agir avec la même main sur le piston de la seringue pour pratiquer l'hydro-dissection ; puis, dans l'étape de crochetage et section du tissu fibreux, le praticien peut maintenir sa prise sur le corps de seringue avec la même main et faire pivoter l'instrument autour du coude pour amener le crochet en prise avec le tissu fibreux à l'avant du tissu fibreux avant d'initier le mouvement de retrait de l'instrument en agissant toujours sur le corps de seringue. En variante, bien entendu, le praticien peut choisir de changer de prise entre l'étape d'avancée du crochet sous le tissu fibreux et l'étape de crochetage et section du tissu fibreux, notamment il peut tenir le corps de seringue pour pratiquer l'hydro-dissection lors de l'étape d'avancée du crochet, puis préférer tenir le corps principal de l'instrument pour faire pivoter l'instrument autour du coude et actionner le mouvement de retrait de l'instrument lors de l'étape de crochetage et section du tissu fibreux.

Dans un mode de réalisation de l'invention, la partie distale de l'instrument de traitement chirurgical a une extrémité distale pointue et tranchante, permettant de pratiquer une incision d'entrée pour l'introduction de l'instrument. Un instrument ayant une telle extrémité distale pointue et tranchante est utilisé lorsque le site interventionnel ne comporte pas de risques de sectionner des nerfs, des vaisseaux ou d'autres structures vulnérables qu'il est important de ne pas sectionner, ce qui est le cas par exemple pour le traitement chirurgical du doigt à ressaut.

Dans un autre mode de réalisation de l'invention, la partie distale de l'instrument de traitement chirurgical a une extrémité distale arrondie. Un instrument ayant une telle extrémité distale arrondie est utilisé lorsque le site interventionnel comporte des risques de sectionner des nerfs, des vaisseaux ou d'autres structures vulnérables qu'il est important de ne pas sectionner, ce qui est le cas par exemple pour le traitement chirurgical du syndrome du canal carpien où les tendons fléchisseurs des doigts et le nerf médian circulant dans le canal carpien doivent être préservés. L'instrument est alors avantageusement associé à une canule d'insertion ayant une extrémité distale pointue et tranchante, permettant de pratiquer une incision d'entrée pour l'introduction de l'instrument sous la peau, l'extrémité distale arrondie de l'instrument étant ensuite déplacée à partir de la canule d'insertion pour avancer sous le tissu fibreux à sectionner sans risque de sectionner des structures vulnérables environnantes.

Selon une caractéristique, le corps principal et la partie distale de l'instrument de traitement chirurgical sont configurés pour coopérer avec une canule d'insertion ayant une extrémité distale pointue et tranchante, la canule d'insertion comportant un corps tubulaire délimitant un logement de réception de la partie distale et une plaque d'appui du coude du corps principal.

Selon une caractéristique, la plaque d'appui de la canule d'insertion a une longueur, prise selon la direction longitudinale du corps tubulaire, supérieure ou égale à la longueur d'un tissu fibreux à sectionner à l'aide de l'instrument de traitement chirurgical. Ainsi, la plaque d'appui fournit un support de longueur suffisante pour que la portion du corps principal en aval du coude puisse glisser systématiquement sur cette plaque d'appui, sans contact direct sur la peau, lors de l'étape d'avancée ou de retrait du crochet en position d'insertion de l'instrument. De plus, cette longueur de la plaque d'appui permet d'éviter toute blessure sur la peau lors du mouvement de retrait de l'instrument, qui pourrait résulter d'un recul de la plaque d'appui si elle était trop courte. A titre d'exemple, dans un mode de réalisation spécialement adapté pour le traitement chirurgical du syndrome du canal carpien, la longueur de la plaque d'appui de la canule d'insertion, prise selon la direction longitudinale du corps tubulaire, supérieure ou égale à la longueur de la zone tendineuse du canal carpien. De préférence, une marge de sécurité est prévue pour la longueur de la plaque d'appui, i.e. la longueur de la plaque d'appui est supérieure, notamment de l'ordre de 10% à 50%, à la longueur du tissu fibreux à sectionner à l'aide de l'instrument de traitement chirurgical.

Selon une caractéristique, la plaque d'appui de la canule d'insertion comporte :
- une partie médiane d'appui du coude de l'instrument de traitement chirurgical, qui est agencée dans le prolongement du corps tubulaire, et
- deux ailes latérales de part et d'autre de la partie médiane, qui sont aptes à être pliées l'une vers l'autre du côté du corps tubulaire.

La partie médiane de la plaque d'appui forme un support permettant non seulement à la portion du corps principal en aval du coude de glisser sans contact direct sur la peau lors de l'étape d'avancée ou de retrait du crochet en position d'insertion de l'instrument, mais aussi au coude de prendre appui lors du pivotement de l'instrument. Les ailes latérales de la plaque d'appui sont quant à elles destinées à être relevées de façon à entourer le corps principal de l'instrument et permettre la préhension du corps principal pour initier le mouvement de retrait de l'instrument générant la section du tissu fibreux.

Selon un aspect non revendiqué de la divulgation, qui peut être considéré indépendamment des caractéristiques décrites ci-dessus, l'invention a pour objet un instrument de traitement chirurgical, notamment pour ténolyses, ténotomies ou neurolyses, comprenant un corps principal et une partie distale, où la partie distale comporte une portion courbe formant un crochet et est munie d'une lame de coupe du côté intérieur du crochet, le corps principal comportant une lumière longitudinale de passage de fluide depuis une extrémité proximale vers une extrémité distale du corps principal, le corps principal de l'instrument comportant également, au voisinage de son extrémité distale, au moins un canal d'injection en connexion fluidique avec la lumière du corps principal, le ou chaque canal d'injection étant configuré pour diriger un jet de fluide radialement vers l'extérieur par rapport au crochet de la partie distale, de préférence sans faire saillie radialement vers l'extérieur par rapport à la partie distale. En particulier, selon un mode de réalisation, le ou chaque canal d'injection est défini dans le prolongement d'une partie supérieure de la lumière du corps principal, et d'une éventuelle chambre de surpression, de manière à diriger un jet de fluide au-dessus du crochet.

L'invention a également pour objet un ensemble selon la revendication 12 comprenant un instrument de traitement chirurgical selon la revendication 1 et une canule d'insertion ayant une extrémité distale pointue et tranchante, la canule d'insertion comportant un corps tubulaire délimitant un logement de réception de la partie distale de l'instrument en position d'insertion, et une plaque d'appui du coude du corps principal en position de coupe.

L'invention a également pour objet un ensemble selon la revendication 13 comprenant un instrument de traitement chirurgical selon la revendication 1 et une seringue dont le corps de seringue est en connexion fluidique avec la lumière du corps principal au niveau de l'extrémité proximale.

Egalement divulgué mais non revendiqué est un ensemble comprenant un instrument de traitement chirurgical et une canule d'insertion dans lequel la plaque d'appui a une longueur, selon la direction longitudinale du corps tubulaire, supérieure ou égale à la longueur d'un tissu fibreux à sectionner.

Selon un autre mode de réalisation, l'invention a aussi pour objet un ensemble selon la revendication 14 comprenant un instrument de traitement chirurgical selon l'une quelconque des revendications 1 à 11 et une canule d'insertion ayant une extrémité distale tranchante, la canule d'insertion comportant un corps tubulaire délimitant un logement de réception de la partie distale, et une plaque d'appui du coude du corps principal, dans lequel la plaque d'appui comporte une partie médiane d'appui du coude dans le prolongement du corps tubulaire et deux ailes latérales de part et d'autre de la partie médiane qui sont aptes à être pliées l'une vers l'autre du côté du corps tubulaire.

Divulguée mais non revendiquée telle qu'elle est une canule d'insertion destinée à coopérer avec un instrument de traitement chirurgical comprenant un corps principal muni d'un coude et une partie distale munie d'un crochet tranchant, la concavité du coude étant tournée du même côté que le crochet tranchant, la canule d'insertion ayant une extrémité distale pointue et tranchante, la canule d'insertion comportant un corps tubulaire délimitant un logement de réception de la partie distale de l'instrument de traitement chirurgical et une plaque d'appui du coude du corps principal de l'instrument de traitement chirurgical, dans laquelle la plaque d'appui a une longueur, prise selon la direction longitudinale du corps tubulaire, supérieure ou égale à la longueur d'un tissu fibreux à sectionner à l'aide de l'instrument de traitement chirurgical. La plaque d'appui fournit ainsi un support de longueur suffisante pour que la portion du corps principal en aval du coude de l'instrument puisse glisser systématiquement sur cette plaque d'appui, sans contact direct sur la peau, lors de l'étape d'avancée ou de retrait du crochet en position d'insertion de l'instrument. De plus, cette longueur de la plaque d'appui permet d'éviter toute blessure sur la peau lors du mouvement de retrait de l'instrument, qui pourrait résulter d'un recul de la plaque d'appui si elle était trop courte.

Selon une caractéristique de la canule d' insertion non revendiquée, la plaque d'appui comporte une partie médiane d'appui du coude dans le prolongement du corps tubulaire, et deux ailes latérales de part et d'autre de la partie médiane qui sont aptes à être pliées l'une vers l'autre du côté du corps tubulaire.

Divulguée mais non comprise dans la portée des revendications est une méthode de traitement chirurgical, comprenant notamment la section d'un tissu fibreux, telle qu'une ténolyse, une ténotomie ou une neurolyse, à l'aide d'un instrument de traitement chirurgical tel que décrit ci-dessus, la méthode comprenant des étapes dans lesquelles :
- on assemble une seringue avec le corps principal de l'instrument, de telle sorte que la seringue est en connexion fluidique avec la lumière du corps principal au niveau de l'extrémité proximale du corps principal ;
- en position d'insertion de l'instrument, dans laquelle la portion du corps principal en aval du coude est parallèle au tissu fibreux à sectionner, on insère la partie distale de l'instrument à travers une incision d'entrée et on engage la partie distale de l'instrument sous le tissu fibreux à sectionner jusqu'à ce que le crochet le dépasse, l'engagement de la partie distale de l'instrument sous le tissu fibreux étant réalisée par hydro-dissection à l'aide d'un fluide contenu dans la seringue et injecté à l'avant de l'extrémité distale de l'instrument, en actionnant le piston de la seringue ;
- à partir de la position du crochet dépassant au-delà du tissu fibreux à sectionner, on pivote l'instrument autour du coude vers une position de coupe, dans laquelle la portion du corps principal en aval du coude est transversale au tissu fibreux à sectionner, de sorte que le crochet est relevé en regard du tissu fibreux à sectionner ;
- on procède à un mouvement de retrait de l'instrument, de telle sorte que le tissu fibreux à sectionner est accroché par le crochet et sectionné par la lame de la partie distale ;
- une fois le tissu fibreux sectionné, on repivote l'instrument autour du coude en sens inverse vers la position d'insertion et on extrait l'instrument à travers l'incision d'entrée.

Selon un mode de réalisation non revendiqué, lorsque la partie distale de l'instrument de traitement chirurgical est insérée à l'aide d'une canule d'insertion, la méthode comprend des étapes dans lesquelles :
- on assemble une seringue avec le corps principal de l'instrument, de telle sorte que la seringue est en connexion fluidique avec la lumière du corps principal au niveau de l'extrémité proximale ;
- on pratique une incision d'entrée à l'aide de l'extrémité distale tranchante de la canule d'insertion ;
- on insère la partie distale de l'instrument dans le logement du corps tubulaire de la canule d'insertion de sorte que le crochet est reçu dans le logement ;
- on insère la partie distale de l'instrument à travers l'incision d'entrée à l'aide de la canule d'insertion, l'instrument étant en position d'insertion ;
- on engage la partie distale de l'instrument sous le tissu fibreux à sectionner en faisant glisser la portion du corps principal en aval du coude sur la plaque d'appui de la canule d'insertion jusqu'à ce que le crochet dépasse au-delà du tissu fibreux à sectionner, l'engagement de la partie distale de l'instrument sous le tissu fibreux étant réalisée par hydro-dissection à l'aide d'un fluide contenu dans la seringue et injecté à l'avant de l'extrémité distale de l'instrument, en actionnant le piston de la seringue ;
- à partir de la position du crochet dépassant au-delà du tissu fibreux à sectionner, on pivote l'instrument autour du coude vers la position de coupe en prenant appui sur la plaque d'appui, de telle sorte que le crochet est relevé en regard du tissu fibreux à sectionner ;
- on procède à un mouvement de retrait de l'instrument en tenant le corps principal à l'aide de la plaque d'appui de la canule d'insertion, de telle sorte que le tissu fibreux à sectionner est accroché par le crochet et sectionné par la lame de la partie distale ;
- une fois le tissu fibreux sectionné, on repivote l'instrument autour du coude en sens inverse vers la position d'insertion, en prenant appui sur la plaque d'appui ;
- on fait glisser la portion du corps principal en aval du coude sur la plaque d'appui de la canule d'insertion jusqu'à ce que le crochet soit reçu dans le logement du corps tubulaire de la canule d'insertion ;
- on extrait l'instrument et la canule d'insertion à travers l'incision d'entrée alors que le crochet est reçu dans le logement du corps tubulaire de la canule d'insertion.

Les caractéristiques et avantages de l'invention apparaîtront dans la description qui va suivre de deux modes de réalisation d'un instrument et d'une méthode de traitement chirurgical non revendiquée, donnée uniquement à titre d'exemple et faite en se référant aux dessins annexés dans lesquels :
- la figure 1 est une coupe d'un instrument de traitement chirurgical conforme à un premier mode de réalisation de l'invention ;
- la figure 2 est une vue à plus grande échelle du détail II de la figure 1 ;
- la figure 3 est une vue à plus grande échelle du détail III de la figure 1 ;
- la figure 4 est une coupe analogue à la figure 1, l'instrument de traitement chirurgical étant associé à une seringue ;
- la figure 5 est une coupe d'une canule d'insertion adaptée pour coopérer avec l'instrument de traitement chirurgical des figures 1 à 4 ;
- la figure 6 est une vue de dessus de la canule d'insertion de la figure 5 ;
- la figure 7 est une coupe de la partie distale d'un instrument de traitement chirurgical conforme à un deuxième mode de réalisation de l'invention.

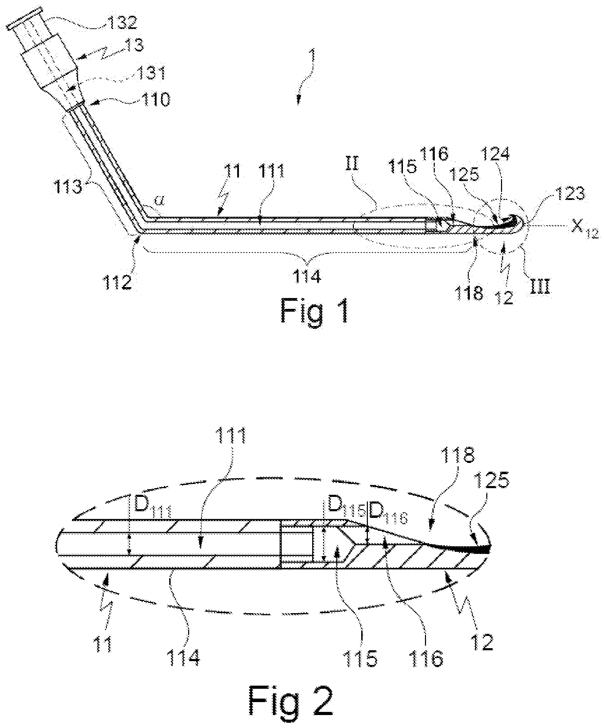

Sur la figure 1 est représenté un instrument de traitement chirurgical 1, de type rétro-couteau à extrémité distale arrondie. A titre d'exemple, l'instrument 1 de ce premier mode de réalisation est destiné au traitement chirurgical du syndrome du canal carpien. L'instrument 1 comprend un corps principal 11, une partie distale 12, et un embout proximal 13 configuré pour coopérer avec une seringue. De manière avantageuse, l'embout 13 est un raccord Luer Lock mâle comprenant un connecteur mâle 132 configuré pour coopérer avec un connecteur 32 complémentaire d'un raccord Luer Lock femelle équipant une seringue 3. L'instrument 1 est ainsi compatible avec des seringues classiques équipées de raccords Luer Lock femelles, qui sont facilement disponibles. La partie distale 12 de l'instrument comporte une portion courbe formant un crochet 124, et est munie d'une lame de coupe 125 du côté intérieur du crochet 124. De plus, le corps principal 11 comporte un coude 112 de concavité tournée du même côté que le crochet 124, qui définit du côté concave un angle de l'ordre de 120° entre une portion amont 113 du corps principal située en amont du coude 112 et une portion avale 114 du corps principal située en aval du coude 112.

Le corps principal 11 de l'instrument comporte également une lumière longitudinale 111 de passage de fluide depuis une extrémité proximale 110 vers une extrémité distale 118 du corps principal 11, qui se poursuit dans l'embout 13 par une lumière correspondante 131. Comme bien visible sur la figure 2, le corps principal 11 comporte, au voisinage de son extrémité distale 118, un canal d'injection 116 qui est en connexion fluidique avec la lumière 111 par l'intermédiaire d'une chambre de surpression 115. Dans ce mode de réalisation, chacun parmi la lumière 111, la chambre de surpression 115 et le canal d'injection 116 est à section circulaire, étant entendu que des sections de formes différentes peuvent être envisagées dans le cadre de l'invention. La chambre de surpression 115 a un diamètre D₁₁₅ supérieur au diamètre D₁₁₁ de la lumière 111 du corps principal et au diamètre D₁₁₆ du canal d'injection 116. Ayant ainsi une section supérieure à celles de la lumière 111 du corps principal et du canal d'injection 116, la chambre de surpression 115 est configurée pour garder en permanence un volume de fluide élevé, de sorte qu'elle est efficace instantanément pour fournir du fluide sous pression au niveau du canal d'injection 116, sans temps de latence. De plus, le volume plus élevé de la chambre de surpression 115 permet de limiter les fuites de fluide incontrôlées au niveau du canal d'injection 116.

Le rapport du diamètre D₁₁₆ du canal d'injection 116 sur le diamètre D₁₁₅ de la chambre de surpression 115 est de l'ordre de 0,45 à 0,5. Cela permet de créer une surpression au niveau du canal d'injection 116, de manière à générer des jets de fluide puissants à partir du canal d'injection. Le rapport du diamètre D₁₁₁ de la lumière 111 du corps principal sur le diamètre D₁₁₅ de la chambre de surpression est de l'ordre de 0,6 à 0,7. Cela permet de maintenir un volume de fluide élevé dans la chambre de surpression 115, avec le double avantage de limiter les fuites de fluide incontrôlées au niveau du canal d'injection 116 et de garantir une réactivité optimale de l'instrument 1 pour fournir du fluide sous pression au niveau du canal d'injection 116, sans temps de latence. En particulier, à titre d'exemple non limitatif, la chambre de surpression 115 a un diamètre D₁₁₅ de 2,2 mm ; la lumière 111 du corps principal 11 a un diamètre D₁₁₁ de 1,4 mm ; le canal d'injection 116 a un diamètre D₁₁₆ de 1,06 mm.

Comme bien visible sur la figure 2, le canal d'injection 116 est défini dans le prolongement d'une partie supérieure de la lumière 111 et d'une partie supérieure de la chambre de surpression 115, de manière à diriger un jet de fluide au-dessus du crochet 124. Ainsi, la redirection des jets de fluide au-dessus du crochet 124 est obtenue sans que le canal 116 fasse saillie radialement vers l'extérieur par rapport à la partie distale 12, ce qui permet de ne pas perturber la progression du crochet 124 lors de son avancée sous le tissu fibreux. La lumière longitudinale 111 du corps principal permet, lors d'une intervention chirurgicale impliquant la section d'un tissu fibreux, d'injecter un fluide en surpression à l'avant et par le dessus du crochet 124 de manière à écarter les parties molles et produire une hydro-dissection lors de la progression de la partie distale 12 de l'instrument sous le tissu fibreux.

Par ailleurs, le coude 112 du corps principal forme un axe de rotation permettant d'opérer une rotation contrôlée de l'instrument 1, de manière à relever le crochet 124 en direction du tissu fibreux pour l'accrocher, avant de le sectionner par un mouvement de retrait de l'instrument 1. Ainsi, l'instrument 1 selon l'invention, dont le corps principal présente à la fois une lumière longitudinale 111 de passage de fluide et un coude 112 de concavité tournée du même côté que le crochet 124, permet d'effectuer avec le même instrument, tenu d'une seule main, et en un seul geste, à la fois l'étape d'avancée du crochet 124 sous le tissu fibreux grâce à l'hydro-dissection, et l'étape de crochetage et section du tissu fibreux grâce au guidage en rotation assuré par le coude 112.

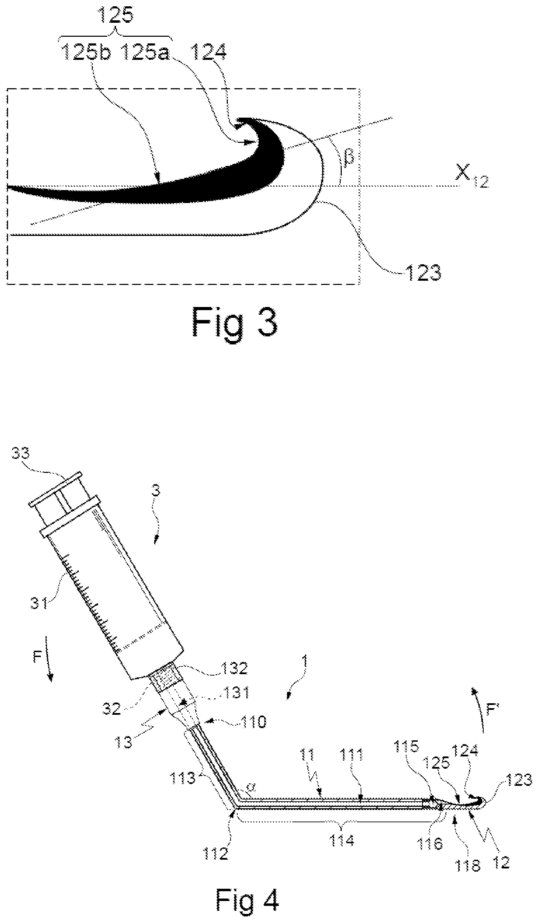

La lame de coupe 125 comprend une partie de lame intérieure 125a, située à l'intérieur dans le fond du crochet 124, et une partie de lame amont 125b, située en amont du crochet 124. Comme bien visible sur la figure 3, la partie de lame amont 125b est inclinée vers l'intérieur du crochet selon un angle β de l'ordre de 25° par rapport à l'axe longitudinal X₁₂ de la partie distale 12. Cette inclinaison de la partie de lame amont 125b permet, une fois le tissu fibreux crocheté à l'aide du crochet 124, d'avoir la partie de lame amont 125b orientée transversalement par rapport au tissu fibreux de manière à pouvoir attaquer la découpe du tissu fibreux en biais plutôt qu'horizontalement. La découpe du tissu fibreux s'opère ainsi au moyen à la fois de la partie de lame intérieure 125a et de la partie de lame amont 125b lors du mouvement de retrait de l'instrument 1. L'inclinaison de la partie de lame amont 125b est particulièrement efficace en association avec le coude 112 du corps principal 11. En effet, le coude 112 permet d'imposer une inclinaison fixe de la partie distale 12 par rapport au tissu fibreux lors du mouvement de retrait, ce qui permet de garantir une prise optimale du tissu fibreux entre la partie de lame intérieure 125a et la partie de lame amont 125b.

A titre d'exemple, dans ce premier mode de réalisation destiné au traitement chirurgical du syndrome du canal carpien, la longueur cumulée de la portion avale 114 du corps principal et de la partie distale 12 est de l'ordre de 75 mm, ce qui correspond à la morphologie du canal carpien. La longueur de la portion amont 113 du corps principal 11 peut quant à elle être modulée pour ajuster le bras de levier souhaité par le chirurgien, et donc la force à appliquer pour sectionner le tissu fibreux lors du mouvement de retrait de l'instrument 1. Dans le cadre du traitement du syndrome du canal carpien, la longueur de la portion amont 113 du corps principal 11 est typiquement de l'ordre de 10 mm à 100 mm.

Au choix, le praticien peut choisir de ne pas changer sa prise sur le corps 31 de la seringue 3 entre l'étape d'avancée du crochet 124 sous le tissu fibreux et l'étape de crochetage et section du tissu fibreux ; ou bien le praticien peut choisir de changer de prise entre l'étape d'avancée du crochet 124 sous le tissu fibreux et l'étape de crochetage et section du tissu fibreux, notamment il peut tenir le corps 31 de la seringue pour pratiquer l'hydro-dissection lors de l'étape d'avancée du crochet 124, puis préférer tenir la portion amont 113 du corps principal 11 de l'instrument pour faire pivoter l'instrument autour du coude 112 et actionner le mouvement de retrait de l'instrument lors de l'étape de crochetage et section du tissu fibreux. La sélection de l'une ou l'autre option peut dépendre, en particulier, de la longueur de la portion amont 113 du corps principal 11.

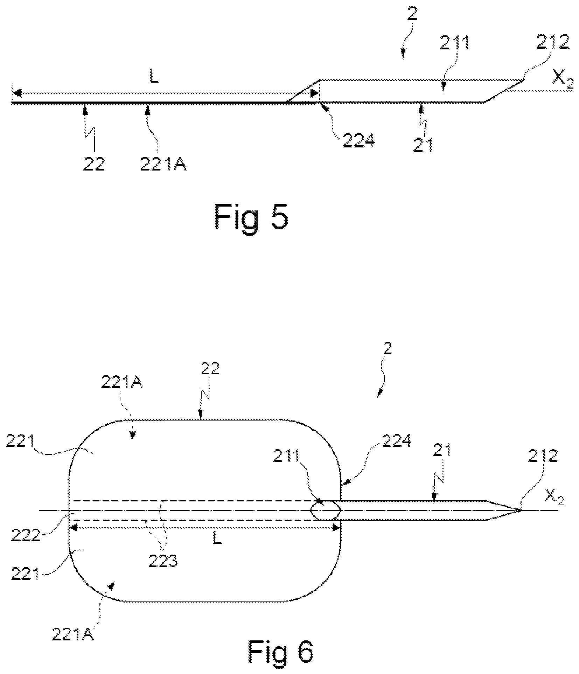

Dans ce premier mode de réalisation, la partie distale 12 de l'instrument 1 a une extrémité distale 123 arrondie. Un instrument 1 ayant une telle extrémité distale arrondie est utilisé notamment lorsque des nerfs, des vaisseaux, ou d'autres structures vulnérables qu'il est important de ne pas sectionner, sont présents au voisinage du tissu fibreux à sectionner, comme c'est le cas par exemple pour une intervention au niveau du canal carpien. Pour son insertion sous la peau, l'instrument 1 est alors avantageusement associé à une canule d'insertion 2 telle que montrée sur les figures 5 et 6, ayant une extrémité distale 212 pointue et tranchante permettant de pratiquer une incision d'entrée pour l'introduction de l'instrument 1. Une fois insérée sous la peau, l'extrémité distale 123 arrondie de l'instrument 1 peut être déplacée à partir de la canule d'insertion 2 pour avancer sous le tissu fibreux à sectionner sans risque de sectionner des structures vulnérables environnantes. Dans ce premier mode de réalisation, à titre d'exemple, l'instrument 1 et la canule d'insertion 2 sont constitués en un métal biocompatible, notamment en acier inoxydable.

La canule d'insertion 2 comporte un corps tubulaire 21 d'axe X₂, qui délimite un logement 211 de réception de la partie distale 12 de l'instrument 1, et une plaque d'appui 22, qui est destinée à recevoir en appui le coude 112 et la partie avale 114 du corps principal 11 de l'instrument 1. La bordure distale 224 de la plaque d'appui 22 forme une limite d'insertion de la canule d'insertion 2 sous la peau.

De plus, la plaque d'appui 22 a une longueur L, prise selon la direction longitudinale du corps tubulaire 21, supérieure ou égale à la longueur du tissu fibreux à sectionner à l'aide de l'instrument 1. Ainsi, on garantit que la plaque d'appui 22 fournit un support de longueur suffisante pour que la portion avale 114 du corps principal 11 puisse glisser systématiquement sans contact direct sur la peau lors de l'étape d'avancée et de retrait du crochet 124 en position d'insertion de l'instrument 1. De plus, une telle longueur de la plaque d'appui 22 permet d'éviter des blessures de la peau lors du mouvement de retrait de l'instrument 1, qui pourraient résulter d'un recul de la plaque d'appui si elle était trop courte. A titre d'exemple, pour une canule d'insertion 2 adaptée pour le traitement chirurgical du syndrome du canal carpien, la longueur L de la plaque d'appui 22 est égale à la longueur de la zone tendineuse du canal carpien additionnée d'une marge de sécurité de 50%, soit de l'ordre de 45 mm.

Comme visible sur la figure 6, la plaque d'appui 22 de la canule d'insertion 2 comporte une partie médiane 222, qui est agencée dans le prolongement du corps tubulaire 21, et deux ailes latérales 221 de part et d'autre de la partie médiane 222, qui sont aptes à être pliées l'une vers l'autre du côté du corps tubulaire 21 selon des lignes de pliage 223 prédéfinies. La partie médiane 222 forme un support permettant à la portion avale 114 du corps principal 11 de glisser sans contact direct sur la peau lors de l'étape d'avancée et de retrait du crochet 124 en position d'insertion de l'instrument 1, et au coude 112 de prendre appui lors du pivotement de l'instrument 1. Les ailes latérales 121 de la plaque d'appui, lorsqu'elles sont relevées de façon à entourer le corps principal 11 de l'instrument 1, permettent la préhension, notamment entre le pouce et l'index du praticien, du corps principal 11 du côté de leur face 221A opposée au corps tubulaire 21 (face inférieure sur la figure 6), afin d'initier le mouvement de retrait de l'instrument 1 générant la section du tissu fibreux.

Un exemple de méthode de traitement chirurgical (non comprise dans la portée des revendications) à l'aide de l'instrument 1, associé à la canule d'insertion 2, comprend des étapes telles que décrites ci-après. A titre d'exemple non limitatif, la méthode décrite ci-dessous peut être mise en œuvre pour le traitement du syndrome du canal carpien, le tissu fibreux à sectionner étant alors le ligament annulaire antérieur du carpe.

Tout d'abord, on assemble une seringue 3, dont le corps de seringue 31 a été préalablement rempli d'un liquide d'hydro-dissection tel qu'une solution saline stérile de type sérum physiologique, avec le corps principal 11 de l'instrument 1, de telle sorte que le corps de seringue 31 est en connexion fluidique avec la lumière 111 du corps principal 11 au niveau de l'extrémité proximale 110. En référence à la figure 4, cette opération est réalisée en connectant le connecteur Luer Lock mâle 132 de l'embout 13 de l'instrument 1 avec le connecteur Luer Lock femelle 32 complémentaire de la seringue 3. On pratique alors une incision d'entrée à l'aide de l'extrémité distale 212 de la canule d'insertion 2 puis, après avoir purgé l'instrument 1 surmonté de la seringue remplie, on insère la partie distale 12 de l'instrument 1 dans le logement 211 du corps tubulaire 21 de la canule d'insertion 2, de telle sorte que le crochet 124 est reçu dans le logement 211. Ce faisant, la partie distale 12 de l'instrument 1 est insérée à travers l'incision d'entrée, l'instrument 1 étant dans une position d'insertion dans laquelle la portion avale 114 du corps principal 11 est parallèle au tissu fibreux à sectionner.

On fait ensuite glisser la portion avale 114 du corps principal 11 sur la partie médiane 222 de la plaque d'appui 22 de la canule d'insertion 2, de manière à engager la partie distale 12 de l'instrument 1 sous le tissu fibreux à sectionner. L'engagement de la partie distale 12 est réalisé en écartant les tissus par hydro-dissection, à l'aide du liquide d'hydro-dissection injecté à l'avant de l'extrémité distale 123 de l'instrument 1 depuis le canal d'injection 116, grâce à l'actionnement du piston 33 de la seringue 3. L'avancée de la partie distale 12 se poursuit jusqu'à ce que le crochet 124 dépasse au-delà du tissu fibreux à sectionner. De manière avantageuse, l'avancée du crochet 124 sous le tissu fibreux à sectionner est contrôlée à l'aide d'une sonde échographique que le praticien tient dans une main, tandis que son autre main actionne à la fois le mouvement d'avancée de l'instrument 1 et le mouvement d'avancée du piston 33 en tenant la seringue 3.

A partir de la position du crochet 124 dépassant au-delà du tissu fibreux à sectionner, on fait pivoter l'instrument 1 dans le sens de la flèche F' de la figure 4, autour du coude 112 posé en appui sur la partie médiane 222 de la plaque d'appui 22, vers une position de coupe dans laquelle la portion avale 114 du corps principal 11 est transversale au tissu fibreux à sectionner. Ainsi, le crochet 124 est relevé en regard du tissu fibreux à sectionner. De manière avantageuse, ce pivotement de l'instrument 1 vers la position de coupe peut être actionné en agissant sur la seringue 3, qui joue le rôle de poignée, dans le sens de la flèche F de la figure 4. En variante, le praticien peut préférer tenir la portion amont 113 du corps principal 11 pour faire pivoter l'instrument 1 autour du coude 112.

On procède alors à un mouvement de retrait de l'instrument 1, en tenant le corps principal 11 à l'aide des ailes latérales 221 de la plaque d'appui 22 de la canule d'insertion 2, pliées l'une vers l'autre du côté du corps tubulaire 21, de telle sorte que le tissu fibreux à sectionner est accroché par le crochet 124 et sectionné par la lame 125. Plus précisément, lors du mouvement de retrait de l'instrument 1, le tissu fibreux est en prise entre la partie de lame intérieure 125a et la partie de lame amont 125b, qui participent toutes les deux à la découpe du tissu fibreux. Une fois le tissu fibreux sectionné, on repivote l'instrument 1 autour du coude 112 en sens inverse, c'est-à-dire dans le sens opposé à la flèche F' de la figure 4, vers la position d'insertion en prenant appui sur la partie médiane 222 de la plaque d'appui 22.

Enfin, on fait glisser la portion avale 114 du corps principal 11 sur la partie médiane 222 de la plaque d'appui 22 de la canule d'insertion 2, jusqu'à ce que le crochet 124 soit reçu dans le logement 211 du corps tubulaire 21 de la canule d'insertion 2, et on extrait l'instrument 1 et la canule d'insertion 2 à travers l'incision d'entrée alors que le crochet 124 est reçu dans le logement 211.

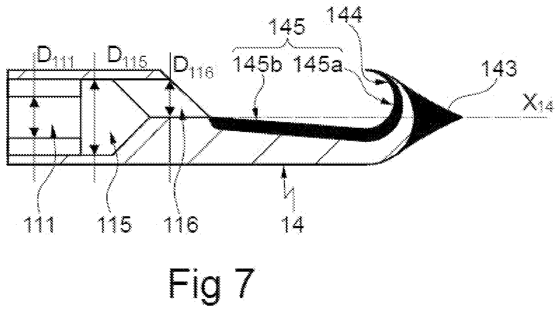

Dans le deuxième mode de réalisation représenté sur la figure 7, les éléments analogues à ceux du premier mode de réalisation portent des références identiques. A titre d'exemple, l'instrument 1 de ce deuxième mode de réalisation est destiné au traitement chirurgical du doigt à ressaut. L'instrument 1 de ce deuxième mode de réalisation diffère de celui du premier mode de réalisation par sa partie distale 14, qui a une extrémité distale 143 pointue et tranchante et une lame de coupe 145 profilée différemment, ainsi que par les diamètres respectifs de la lumière 111, la chambre de surpression 115 et le canal d'injection 116.

De manière analogue au premier mode de réalisation, la partie distale 14 comporte une portion courbe formant un crochet 144, et est munie d'une lame de coupe 145 du côté intérieur du crochet 144. L'extrémité distale 143 pointue et tranchante a l'avantage de permettre de pratiquer une incision d'entrée pour l'introduction de l'instrument 1 directement avec l'instrument 1 lui-même, sans avoir à recourir à une canule d'insertion complémentaire. Toutefois, un instrument 1 ayant une telle extrémité distale pointue et tranchante n'est utilisé que lorsque le site interventionnel ne comporte pas de risques de sectionner des nerfs, des vaisseaux ou d'autres structures vulnérables, ce qui est le cas par exemple pour le traitement chirurgical du doigt à ressaut. L'instrument 1 du deuxième mode de réalisation présente par ailleurs les mêmes avantages que celui du premier mode de réalisation.

Dans ce deuxième mode de réalisation, à titre d'exemple, l'instrument 1 est constitué en un métal biocompatible, notamment en acier inoxydable. Comme dans le premier mode de réalisation, le rapport du diamètre D₁₁₆ du canal d'injection 116 sur le diamètre D₁₁₅ de la chambre de surpression 115 est de l'ordre de 0,45 à 0,5, et le rapport du diamètre D₁₁₁ de la lumière 111 du corps principal sur le diamètre D₁₁₅ de la chambre de surpression est de l'ordre de 0,6 à 0,7. En particulier, à titre d'exemple non limitatif, la chambre de surpression 115 a un diamètre D₁₁₅ de 1,6 mm ; la lumière 111 du corps principal 11 a un diamètre D₁₁₁ de 1 mm ; le canal d'injection 116 a un diamètre D₁₁₆ de 0,8 mm.

Comme dans le premier mode de réalisation, la lame de coupe 145 comprend une partie de lame intérieure 145a, située à l'intérieur dans le fond du crochet 144, et une partie de lame amont 145b, située en amont du crochet 144. Il est à noter que, dans le deuxième mode de réalisation, la partie de lame amont 145b est légèrement inclinée vers l'extérieur du crochet 144 par rapport à l'axe longitudinal X₁₄ de la partie distale 14, au lieu d'être inclinée vers l'intérieur du crochet comme dans le premier mode de réalisation. Bien entendu, en variante, la partie de lame amont 145b peut être inclinée vers l'intérieur du crochet, en particulier selon un angle compris entre 5° et 30°, par rapport à l'axe longitudinal de la partie distale 14, pour permettre, une fois le tissu fibreux crocheté à l'aide du crochet, d'avoir la partie de lame amont 145b orientée transversalement par rapport au tissu fibreux de sorte qu'elle peut attaquer la découpe du tissu fibreux en biais plutôt qu'horizontalement.

Un exemple de méthode de traitement chirurgical (non comprise dans la portée des revendications) à l'aide de l'instrument 1 du deuxième mode de réalisation, sans utiliser de canule d'insertion, comprend des étapes telles que décrites ci-après. A titre d'exemple non limitatif, la méthode décrite ci-dessous peut être mise en œuvre pour le traitement du doigt à ressaut, le tissu fibreux à sectionner étant alors une poulie entourant un tendon fléchisseur.

Tout d'abord, on assemble une seringue 3, dont le corps de seringue 31 a été préalablement rempli d'un liquide d'hydro-dissection tel qu'une solution saline stérile de type sérum physiologique, avec le corps principal 11 de l'instrument 1, de telle sorte que le corps de seringue 31 est en connexion fluidique avec la lumière 111 du corps principal 11 au niveau de l'extrémité proximale 110.

Après avoir purgé l'instrument 1 surmonté de la seringue remplie, on pratique ensuite une incision d'entrée à l'aide de l'extrémité distale 143 pointue et tranchante et, en position d'insertion de l'instrument 1 dans laquelle la portion avale 114 du corps principal 111 est parallèle au tissu fibreux à sectionner, on insère la partie distale 14 de l'instrument à travers l'incision d'entrée.

On fait ensuite avancer la portion avale 114 du corps principal 11, de manière à engager la partie distale 14 de l'instrument 1 sous le tissu fibreux à sectionner. L'engagement de la partie distale 14 est réalisé en écartant les tissus par hydro-dissection, à l'aide du liquide d'hydro-dissection injecté à l'avant de l'extrémité distale 143 de l'instrument 1 depuis le canal d'injection 116, grâce à l'actionnement du piston 33 de la seringue 3. L'avancée de la partie distale 14 se poursuit jusqu'à ce que le crochet 144 dépasse au-delà du tissu fibreux à sectionner. De manière avantageuse, l'avancée du crochet 144 sous le tissu fibreux à sectionner est contrôlée à l'aide d'une sonde échographique que le praticien tient dans une main, tandis que son autre main actionne à la fois le mouvement d'avancée de l'instrument 1 et le mouvement d'avancée du piston 33 en tenant la seringue 3.

A partir de la position du crochet 144 dépassant au-delà du tissu fibreux à sectionner, on fait pivoter l'instrument 1 autour du coude 112 dans un sens similaire à celui de la flèche F' de la figure 4, vers une position de coupe dans laquelle la portion avale 114 du corps principal 11 est transversale au tissu fibreux à sectionner. Ainsi, le crochet 144 est relevé en regard du tissu fibreux à sectionner.

On procède alors à un mouvement de retrait de l'instrument 1, notamment en tenant le corps de seringue 31 ou le corps principal 11, de telle sorte que le tissu fibreux à sectionner est accroché par le crochet 144 et sectionné par la lame 145. Une fois le tissu fibreux sectionné, on repivote l'instrument 1 autour du coude 112 en sens inverse, c'est-à-dire dans le sens opposé à celui de la flèche F' de la figure 4, vers la position d'insertion, et on extrait l'instrument 1 à travers l'incision d'entrée.

Comme il ressort des exemples précédents, quel que soit le mode de réalisation, grâce à la présence de la lumière 111 et du coude 112 du corps principal, un instrument de traitement chirurgical selon l'invention permet d'effectuer avec le même instrument, tenu d'une seule main, et en un seul geste, à la fois l'étape d'avancée du crochet 124, 144 sous le tissu fibreux grâce à l'hydro-dissection, et l'étape de crochetage et section du tissu fibreux grâce au guidage en rotation assuré par le coude 112, sans nécessiter d'inciser préalablement la peau avec un bistouri pour introduire l'instrument. De manière très avantageuse, un instrument de traitement chirurgical selon l'invention est compatible avec un monitoring à l'aide d'un appareil, par exemple une sonde échographique, tenu dans l'autre main que celle qui actionne l'instrument. Un instrument de traitement chirurgical selon l'invention permet plus généralement de pratiquer des interventions mieux contrôlées, ce qui limite les douleurs post-opératoires pour le patient et la durée d'arrêt d'activité.

L'invention n'est pas limitée aux exemples décrits et représentés.

En particulier, dans les exemples précédents, l'invention a été décrite pour la section de tissus fibreux du poignet ou du doigt, notamment dans le cadre du traitement du syndrome du canal carpien ou du doigt à ressaut. Toutefois, un instrument de traitement chirurgical et une canule d'insertion selon l'invention peuvent être utilisés pour la section de tous tissus fibreux, notamment de tissus fibreux de la main, de l'épaule, du genou, de la cheville, du pied, les dimensions de l'instrument et de la canule d'insertion étant alors adaptées au cas par cas pour correspondre à la longueur et à l'environnement du tissu fibreux à sectionner.

De manière générale, la géométrie d'un instrument de traitement chirurgical selon l'invention peut être différente de celles décrites précédemment. En particulier : la longueur cumulée de la portion du corps principal en aval du coude et de la partie distale, si elle est sensiblement figée pour une application donnée, tend à varier d'une application à une autre ; la longueur de la portion du corps principal en aval du coude peut être modulée de manière à ajuster le bras de levier et donc la force à appliquer par le praticien pour sectionner le tissu fibreux lors du mouvement de retrait de l'instrument ; ou encore les diamètres respectifs de la lumière du corps principal, de la chambre de surpression et du ou des canaux d'injection peuvent être ajustés pour optimiser l'hydro-dissection. Un instrument de traitement chirurgical et une canule d'insertion selon l'invention peuvent également être constitués en tous matériaux adaptés à leur fonction, notamment en métal et/ou polymère biocompatibles. L'utilisation d'un matériau amagnétique, notamment un polymère, est avantageux pour permettre un contrôle par IRM (Imagerie par Résonance Magnétique) d'une intervention mettant en jeu un instrument de traitement chirurgical et/ou et d'une canule d'insertion selon l'invention.

## Revendications

1. Instrument de traitement chirurgical (1), notamment pour ténolyses, ténotomies ou neurolyses, comprenant :
- un corps principal (11) comportant une lumière longitudinale (111) de passage de fluide depuis une extrémité proximale (110) vers une extrémité distale (118) du corps principal (11), et
- une partie distale (12 ; 14), comportant une portion courbe formant un crochet (124; 144) et étant munie d'une lame de coupe (125 ; 145) du côté intérieur du crochet (124 ; 144),
le corps principal (11) comportant en outre un coude (112) de concavité tournée du même côté que le crochet (124 ; 144),
**caractérisé en ce que** le corps principal (11) comporte, au voisinage de son extrémité distale (118) :
- une chambre de surpression (115), et
- un canal d'injection (116),
le canal d'injection (116) étant en connexion fluidique avec la lumière (111) par l'intermédiaire de la chambre de surpression (115), le canal d'injection (116) étant destiné à diriger un jet de fluide au-dessus du crochet (124), la chambre de surpression (115) étant configurée pour fournir du fluide sous pression au niveau du canal d'injection (116).

2. Instrument de traitement chirurgical selon la revendication **1,** dans lequel le ou chaque canal d'injection (116) est configuré pour diriger un jet de fluide radialement vers l'extérieur par rapport au crochet (124 ; 144) de la partie distale (12 ; 14).

3. Instrument de traitement chirurgical selon la revendication **2,** dans lequel la chambre de surpression (115) est de section transversale (D₁₁₅) supérieure à la section transversale (D₁₁₁) de la lumière (111) du corps principal (11) et à la section transversale (D₁₁₆) de chaque canal d'injection (116).

4. Instrument de traitement chirurgical selon l'une quelconque des revendications **2** ou **3,** dans lequel le ou chaque canal d'injection (116) est défini dans le prolongement d'une partie supérieure de la lumière (111) du corps principal, et d'une partie supérieure de la chambre de surpression (115) lorsqu'elle est présente, de manière à diriger un jet de fluide au-dessus du crochet (124 ; 144).

5. Instrument de traitement chirurgical selon la revendication **3,** dans lequel le rapport de la section transversale (D₁₁₁) de la lumière (111) du corps principal (11) sur la section transversale (D₁₁₅) de la chambre de surpression (115) est inférieur ou égal à 0,7, de préférence compris entre 0,6 et 0,7, et dans lequel, pour le ou chaque canal d'injection (116), le rapport de la section transversale (D₁₁₆) du canal d'injection (116) sur la section transversale (D₁₁₅) de la chambre de surpression (115) est inférieur ou égal à 0,5, de préférence compris entre 0,45 et 0,5.

6. Instrument de traitement chirurgical selon l'une quelconque des revendications précédentes, dans lequel le coude (112) définit du côté concave un angle (α) compris entre 90° et 160°, de préférence de l'ordre de 120°, entre une portion (113) du corps principal (11) en amont du coude (112) et une portion (114) du corps principal (11) en aval du coude (112).

7. Instrument de traitement chirurgical selon l'une quelconque des revendications précédentes, dans lequel la lame de coupe (125 ; 145) du crochet (124 ; 144) comporte une partie de lame intérieure (125a ; 145a) à l'intérieur du crochet (124 ; 144) et une partie de lame amont (125b ; 145b) en amont du crochet (124 ; 144), la partie de lame amont (125b ; 145b) étant inclinée vers l'intérieur du crochet, notamment selon un angle (β) compris entre 5° et 30°, par rapport à l'axe longitudinal (X₁₂ ; X₁₄) de la partie distale (12 ; 14).

8. Instrument de traitement chirurgical selon l'une quelconque des revendications précédentes, comprenant un embout (13) configuré pour coopérer avec une seringue (3) de manière à mettre le corps de seringue (31) en connexion fluidique avec la lumière (111) du corps principal (11) au niveau de l'extrémité proximale (110).

9. Instrument de traitement chirurgical selon l'une quelconque des revendications **1** à **8,** dans lequel la partie distale (14) a une extrémité distale (143) tranchante.

10. Instrument de traitement chirurgical selon l'une quelconque des revendications **1** à **8,** dans lequel la partie distale (12) a une extrémité distale (123) arrondie.

11. Instrument de traitement chirurgical selon la revendication **10,** dans lequel le corps principal (11) et la partie distale (12) sont configurés pour coopérer avec une canule d'insertion (2) ayant une extrémité distale (212) tranchante, la canule d'insertion (2) comportant un corps tubulaire (21) délimitant un logement (211) de réception de la partie distale (12) et une plaque (22) d'appui du coude (112) du corps principal (11).

12. Ensemble comprenant un instrument de traitement chirurgical (1) selon l'une quelconque des revendications **1** à **11** et une canule d'insertion (2) ayant une extrémité distale (212) tranchante, la canule d'insertion (2) comportant un corps tubulaire (21) délimitant un logement (211) de réception de la partie distale (12), et une plaque (22) d'appui du coude (112) du corps principal (11).

13. Ensemble comprenant un instrument de traitement chirurgical (1) selon l'une quelconque des revendications **1** à **11** et une seringue (3) en connexion fluidique avec la lumière (111) du corps principal (11) au niveau de l'extrémité proximale (110).

14. Ensemble selon la revendication 12, dans lequel la plaque d'appui (22) comporte une partie médiane (222) d'appui du coude (112) dans le prolongement du corps tubulaire (21) et deux ailes latérales (221) de part et d'autre de la partie médiane (222) qui sont aptes à être pliées l'une vers l'autre du côté du corps tubulaire (21).

## Patentansprüche

1. Chirurgisches Behandlungsinstrument (1), insbesondere für Tenolysen, Tenotomien oder Neurolysen, umfassend:
- einen Hauptkörper (11), der ein Längslumen (111) für den Fluiddurchgang von einem proximalen Ende (110) zu einem distalen Ende (118) des Hauptkörpers (11) aufweist, und
- einen distalen Teil (12; 14), der einen gebogenen Abschnitt aufweist, der einen Haken (124; 144) bildet, und auf der Innenseite des Hakens (124; 144) mit einer Schneidklinge (125; 145) versehen ist,
wobei der Hauptkörper (11) ferner eine Krümmung (112) mit einer Konkavität aufweist, die auf die gleiche Seite wie der Haken (124; 144) gedreht ist,
**dadurch gekennzeichnet, dass** der Hauptkörper (11) in der Nähe seines distalen Endes (118) Folgendes aufweist:
- eine Überdruckkammer (115), und
- einen Injektionskanal (116),
wobei der Injektionskanal (116) über die Überdruckkammer (115) mit dem Lumen (111) fluidisch verbunden ist, wobei der Injektionskanal (116) dazu bestimmt ist, einen Fluidstrahl über den Haken (124) zu leiten, wobei die Überdruckkammer (115) dazu eingerichtet ist, das Druckfluid auf der Höhe des Injektionskanals (116) bereitzustellen.

2. Chirurgisches Behandlungsinstrument nach Anspruch **1,** wobei der oder jeder Injektionskanal (116) dazu eingerichtet ist, einen Fluidstrahl in Bezug auf den Haken (124; 144) des distalen Teils (12; 14) radial nach außen zu leiten.

3. Chirurgisches Behandlungsinstrument nach Anspruch **2,** wobei die Überdruckkammer (115) einen Querschnitt (D₁₁₅) hat, der größer ist als der Querschnitt (D₁₁₁) des Lumens (111) des Hauptkörpers (11) und der Querschnitt (D₁₁₆) jedes Injektionskanals (116).

4. Chirurgisches Behandlungsinstrument nach einem der Ansprüche **2** oder **3,** wobei der oder jeder Injektionskanal (116) in Verlängerung eines oberen Teils des Lumens (111) des Hauptkörpers und eines oberen Teils der Überdruckkammer (115), wenn vorhanden, so definiert ist, dass ein Fluidstrahl über den Haken (124; 144) geleitet wird.

5. Chirurgisches Behandlungsinstrument nach Anspruch **3,** wobei das Verhältnis des Querschnitts (D₁₁₁) des Lumens (111) des Hauptkörpers (11) zum Querschnitt (D₁₁₅) der Überdruckkammer (115) kleiner oder gleich 0,7 ist, vorzugsweise zwischen 0,6 und 0,7 liegt, und wobei für den oder jeden Injektionskanal (116) das Verhältnis des Querschnitts (D₁₁₆) des Injektionskanals (116) zum Querschnitt (D₁₁₅) der Überdruckkammer (115) kleiner oder gleich 0,5 ist, vorzugsweise zwischen 0,45 und 0,5 liegt.

6. Chirurgisches Behandlungsinstrument nach einem der vorhergehenden Ansprüche, wobei die Krümmung (112) auf der konkaven Seite einen Winkel (α) zwischen 90° und 160°, vorzugsweise im Bereich von 120°, zwischen einem Abschnitt (113) des Hauptkörpers (11) vor der Krümmung (112) und einem Abschnitt (114) des Hauptkörpers (11) nach der Krümmung (112) definiert.

7. Chirurgisches Behandlungsinstrument nach einem der vorhergehenden Ansprüche, wobei die Schneidklinge (125; 145) des Hakens (124; 144) einen inneren Klingenteil (125a; 145a) im Inneren des Hakens (124; 144) und einen vorgelagerten Klingenteil (125b; 145b) vor dem Haken (124; 144) aufweist, wobei der vorgelagerte Klingenteil (125b; 145b) zum Inneren des Hakens hin geneigt ist, insbesondere in einem Winkel (β) zwischen 5° und 30°, in Bezug auf die Längsachse (X₁₂; X₁₄) des distalen Teils (12; 14).

8. Chirurgisches Behandlungsinstrument nach einem der vorhergehenden Ansprüche, umfassend eine Spitze (13), die dazu eingerichtet ist, mit einer Spritze (3) zusammenzuwirken, um den Spritzenkörper (31) mit dem Lumen (111) des Hauptkörpers (11) am proximalen Ende (110) fluidisch zu verbinden.

9. Chirurgisches Behandlungsinstrument nach einem der Ansprüche **1** bis **8,** wobei der distale Teil (14) ein scharfkantiges distales Ende (143) hat.

10. Chirurgisches Behandlungsinstrument nach einem der Ansprüche **1** bis **8,** wobei der distale Teil (12) ein abgerundetes distales Ende (123) hat.

11. Chirurgisches Behandlungsinstrument nach Anspruch **10,** wobei der Hauptkörper (11) und der distale Teil (12) dazu eingerichtet sind, mit einer Einführkanüle (2) zusammenzuwirken, die ein scharfkantiges distales Ende (212) hat, wobei die Einführkanüle (2) einen röhrenförmigen Körper (21) aufweist, der ein Aufnahmegehäuse (211) für den distalen Teil (12) und eine Auflageplatte (22) für die Krümmung (112) des Hauptkörpers (11) begrenzt.

12. Baugruppe, umfassend ein chirurgisches Behandlungsinstrument (1) nach einem der Ansprüche **1** bis **11** und eine Einführkanüle (2), die ein scharfkantiges distales Ende (212) hat, wobei die Einführkanüle (2) einen röhrenförmigen Körper (21) aufweist, der ein Aufnahmegehäuse (211) für den distalen Teil (12) und eine Auflageplatte (22) für die Krümmung (112) des Hauptkörpers (11) begrenzt.

13. Baugruppe, umfassend ein chirurgisches Behandlungsinstrument (1) nach einem der Ansprüche **1** bis **11** und eine Spritze (3), die mit dem Lumen (111) des Hauptkörpers (11) am proximalen Ende (110) fluidisch verbunden ist.

14. Baugruppe nach Anspruch 12, wobei die Auflageplatte (22) einen mittleren Teil (222) zur Auflage der Krümmung (112) in der Verlängerung des röhrenförmigen Körpers (21) und zwei seitliche Flügel (221) auf beiden Seiten des mittleren Teils (222) aufweist, die auf der Seite des röhrenförmigen Körpers (21) zueinander gebogen werden können.

## Claims

1. Surgical treatment instrument (1), in particular for tenolysis, tenotomy or neurolysis, comprising:
- a main body (11) comprising a longitudinal lumen (111) for passage of fluid from a proximal end to a distal end (110) to a distal end (118) of the main body (11), and
- a distal part (12; 14) including a curved portion forming a hook (124; 144) and being provided with a cutting blade (125; 145) on the inner side of the hook (124; 144),
the main body (11) further comprising an elbow (112) presenting a concavity facing the same side as the hook (124; 144),
**characterised in that** the main body has, in the vicinity of its distal end (118):
- an over-pressure chamber (115),
- an injection channel (116),
the injection channel (116) being in fluidic connection with the lumen (111) via the over-pressure chamber (115), the injection channel (116) being configured to direct a fluid stream above the hook (124), the over-pressure chamber (115) being configured to deliver pressurised fluid to the injection channel (116).

2. Surgical treatment instrument according to claim **1,** wherein the or each injection channel (116) is configured to direct a stream of fluid radially outwardly from the hook (124; 144) of the distal portion (12; 14).

3. Surgical treatment instrument according to claim **2,** wherein the over-pressure chamber (115) has a cross-sectional area (D₁₁₅) greater than the cross-sectional area (D₁₁₁) of the lumen (111) of the main body (11) and the cross-sectional area (D₁₁₆) of each injection channel (116).

4. Surgical treatment instrument according to any one of claims **2** or **3,** wherein the or each injection channel (116) is defined as an extension of an upper portion of the lumen (111) of the main body, and an upper portion of the over-pressure chamber (115) where present, so as to direct a stream of fluid above the hook (124; 144).

5. Surgical treatment instrument according to claim **3,** wherein the ratio of the cross-sectional area (D₁₁₁) of the lumen (111) of the main body (11) to the cross-sectional area (D₁₁₅) of the over-pressure chamber (115) is less than or equal to 0.7, preferably between 0, 6 to 0.7, and wherein for the or each injection channel (116) the ratio of the cross-sectional area (D₁₁₆) of the injection channel (116) to the cross-sectional area (D₁₁₅) of the over-pressure chamber (115) is less than or equal to 0.5, preferably between 0.45 and 0.5.

6. Surgical treatment instrument according to any one of the preceding claims, wherein the elbow (112) defines on the concave side an angle (α) of between 90° and 160°, preferably of the order of 120°, between a portion (113) of the main body (11) upstream of the elbow (112) and a portion (114) of the main body (11) downstream of the elbow (112).

7. Surgical treatment instrument according to any one of the preceding claims, wherein the cutting blade (125; 145) of the hook (124; 144) comprises an inner blade portion (125a; 145a) inside the hook (124; 144) and an upstream blade portion (125b ; 145b) upstream of the hook (124; 144), the upstream blade portion (125b; 145b) being inclined towards the inside of the hook, in particular at an angle (β) of between 5° and 30°, relative to the longitudinal axis (X12; X14) of the distal portion (12; 14).

8. Surgical treatment instrument according to any one of the preceding claims, comprising a tip (13) configured to cooperate with a syringe (3) so as to bring the syringe body (31) into fluidic connection with the lumen (111) of the main body (11) at the proximal end (110).

9. The surgical treatment instrument according to any of claims **1** to **8,** wherein the distal portion (14) has a sharp distal end (143).

10. Surgical treatment instrument according to any one of claims **1** to **8,** wherein the distal portion (12) has a rounded distal end (123).

11. Surgical treatment instrument according to claim **10,** wherein the main body (11) and the distal portion (12) are configured to cooperate with an insertion cannula (2) having a sharp distal end (212), the insertion cannula (2) comprising a tubular body (21) delimiting a housing (211) for receiving the distal portion (12) and a support plate (22) for the elbow (112) of the main body (11).

12. Assembly comprising a surgical treatment instrument (1) according to any one of claims **1** to **11** and an insertion cannula (2) having a sharp distal end (212), the insertion cannula (2) comprising a tubular body (21) delimiting a housing (211) for receiving the distal part (12), and a plate (22) for supporting the elbow (112) of the main body (11).

13. Assembly comprising a surgical treatment instrument (1) according to any of claims **1** to **11** and a syringe (3) in fluidic connection with the lumen (111) of the main body (11) at the proximal end (110).

14. Assembly according to claim 12, wherein the support plate (22) comprises a median part (222) for supporting the elbow (112) in the extension of the tubular body (21) and two lateral wings (221) on either side of the median part (222), which are capable of being folded towards each other on the side of the tubular body (21).
